# EUROPEAN PATENT SPECIFICATION

(11) **EP 0 920 865 B1**
(45) Date of publication and mention of the grant of the patent: **14.01.2004**
(21) Application number: 99200247.7
(22) Date of filing: 03.09.1993
(51) Int. Cl.: A61K 31/445, A61K 45/06, A61P 37/08

(54) **Pharmaceutical compositions containing Norastemizole**
Pharmazeutische Zusammensetzungen enthaltend Norastemizol.
Compositions pharmaceutiques contenant du Astemizole

(30) Priority: 03.09.1992 US 940054
(43) Date of publication of application: 09.06.1999
(62) Divisional of application: 93921359.1
(73) Proprietor: Sepracor Inc., Marlborough, Massachusetts 01752 (US)
(72) Inventor: Woosley, Raymond L., Washington, DC 20016 (US); Aberg, A.K. Gunnar, Sarasota, Florida 34242-1816 (US)
(74) Representative: Jump, Timothy John Simon

(56) References cited:
- EP-A- 0 232 937
- KAMEI, C. ET AL: "Antiallergic effects of major metabolites of Astemizole in rats and guinea pigs" ARZNEIMITTEL FORSCHUNG, vol. 41, no. II, September 1991, page 932-36 XP002098847

## Description

This invention relates to novel pharmaceutical compositions containing norastemizole. These compositions possess potent antihistaminic activity and are useful in treating allergic rhinitis, asthma and other allergic disorders while avoiding adverse effects associated with the administration of other antihistamines, such as astemizole, including but not limited to cardiac arrhythmias, drowsiness, nausea, fatigue, weakness and headache. Also, these compositions, in combination with non-steroidal anti-inflammatory agents or other non-narcotic analgesics, are useful for the treatment of cough, cold, cold-like, and/or flu symptoms and the discomfort, headache, pain, fever, and general malaise associated therewith. The aforementioned combinations may optionally include one or more other active components including a decongestant, cough suppressant/antitussive, or expectorant.

Additionally, these novel pharmaceutical compositions containing norastemizole are useful in treating motion sickness, vertigo, diabetic retinopathy, small vessel complications due to diabetes and such other conditions as may be related to the activity of these derivatives as antagonists of the H-1 histamine receptor while avoiding the adverse effects associated with the administration of other antihistamines, such as astemizole.

Also disclosed are methods for treating the above-described conditions in a human while avoiding the adverse effects that are associated with the administration of other antihistamines, such as astemizole, by administering the aforementioned pharmaceutical compositions containing norastemizole to said human.

The active compound of these compositions and methods is a metabolic derivative of astemizole. Chemically, this derivative is norastemizole. This compound is described in Kamei et al., Arzneimittel-Forschung/Drug Research, 41: 932-36 (1991).

Astemizole is an antagonist of the H-1 histamine receptor protein. Histamine receptor proteins occur in two well-identified forms in tissues as H-1 and H-2 receptors. The H-1 receptors are those that mediate the response antagonized by conventional antihistamines. H-1 receptors are present, for example, in the ileum, the skin, and the bronchial smooth muscle of man and other mammals. Astemizole antagonizes the effect of histamine in the guinea pig isolated ileum, suppresses histamine-induced whealing in the skin of guinea pigs, and protects against histamine induced bronchoconstriction in the guinea pig.

Through H-2 receptor-mediated responses, histamine stimulates gastric acid secretion in mammals and the chronotropic effect in isolated mammalian atria. Astemizole has no effect on histamine-induced gastric acid secretion, nor does it alter the chronotropic effect of histamine on atria. Thus, astemizole has no apparent effect on the H-2 histamine receptor.

Astemizole is well absorbed but is extensively metabolized. see Uchiyama et al., Pharmacometrics, 40: 77-93 (1990). Three main metabolites have been identified, and all of the metabolites are reported to have antihistaminic activity. See Kamei et al., Arzneimittel-Forschung/Drug Research, 41: 932-36 (1991).

On the basis of its antihistaminic activity, researchers evaluated the pharmacological effects of astemizole in man. Clinical trials of efficacy indicated that astemizole is an effective H-1 antagonist. See Howarth, Clin. Exp. Allergy, 20 (Suppl. 2): 31-41 (1990).

Weintraub et al., Hosp. Formul., 22: 918-27 (1987) describes clinical efficacy of astemizole in the treatment of both seasonal and perennial allergies. It has also been suggested that astemizole would be useful for the treatment of asthma.

Astemizole may also be useful for the treatment of motion sickness and vertigo. Some antihistamines have been found to be effective for the prophylaxis and treatment of motion sickness. See Wood, Drugs, 17: 471-479 (1979). Some antihistamines have also proven useful for treating vestibular disturbances, such as Meniere's disease, and in other types of vertigo. See Cohen et al., Archives of Neurology, 27: 129-135 (1972).

In addition, astemizole may be useful in the treatment of diabetic retinopathy and other small vessel disorders associated with diabetes mellitus. In tests on rats with streptozocin-induced diabetes, treatment by antihistamines prevented the activation of retinal histamine receptors which have been implicated in the development of diabetic retinopathy. The use of antihistamines to treat retinopathy and small vessel disorders associated with diabetes mellitus is disclosed in U.S. Patent No. 5,019,591.

Many antihistamines cause somewhat similar adverse effects. These adverse effects include but are not limited to sedation, gastrointestinal distress, dry mouth, and constipation or diarrhea. Astemizole has been found to cause relatively less sedation as compared with other antihistamines. See Weintraub et al., Hosp. Formul., 22: 918-27 (1987).

However, the administration of astemizole to a human has been found to cause other adverse effects. These adverse effects include but are not limited to cardiac arrhythmias, including ventricular tachyarrhythmias, torsades de pointes, and ventricular fibrillation. Recently, clinical practitioners have noted an increase in the occurrence of these cardiac arrhythmias upon co-administration of astemizole with other drugs such as ketoconazole and erythromycin or upon overdose of astemizole. See Knowles, Canadian Journal Hosp. Pharm., 45: 33,37 (1992); Craft, British Medical Journal, 292: 660 (1986); Simons et al., Lancet, 2: 624 (1988); and Unknown, Side-effects or Drugs Annual, 12: 142 and 14: 135. An additional unwanted side effect of astemizole is appetite stimulation and weight gain in patients taking the drug for various indications. See Krstenansky et al., Drug Intell. Clin. Pharm., 21: 947-53 (1987).

Thus, it would be particularly desirable to find a compound with the advantages of astemizole which would not have the aforementioned disadvantages.

It has now been discovered that norastemizole (a metabolic derivative of astemizole) is an effective antihistamine. It has also been discovered that pharmaceutical compositions containing norastemizole are useful in treating allergic disorders and such other conditions as may be related to the composition's activity as an antihistamine, including but not limited to allergic rhinitis, solar urticaria, and symptomatic dermographism.

Furthermore, it has now also been discovered that norastemizole is useful in treating asthma. Also, norastemizole is useful for the treatment of motion sickness and vertigo and in treating such disorders as retinopathy and small vessel disorders associated with diabetes mellitus.

It has also been discovered that norastemizole, in combination with non-steroidal anti-inflammatory agents or other non-narcotic analgesics, is useful for the treatment of cough, cold, cold-like and/or flu symptoms and the discomfort, pain, headache, fever, and general malaise associated therewith. The use of pharmaceutical compositions of the invention, containing (1) norastemizole and (2) non-narcotic analgesics or non-steroidal anti-inflammatory agents such as aspirin, acetaminophen or ibuprofen, may optionally include one or more other active components including a decongestant (such as pseudoephedrine), a cough suppressant/antitussive (such as dextromethorphan) or an expectorant (such as guaifenesin).

Accordingly and in a first aspect, the present invention provides a pharmaceutical composition comprising norastemizole, the structure of which is depicted by formula I: 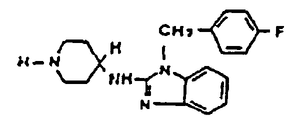 or a pharmaceutically acceptable salt thereof, for use in an anti-histaminic treatment which does not induce any significant cardiac arrhythmia, comprising administering a therapeutically effective amount of the compound of formula I to a human patient.

Prior to the present invention, those skilled in the art would have expected compounds of formula I to have induced a form of cardiac arrhythmia known as torsades de pointes (as reported by Brian P. Monahan et. al., in JAMA, 5th Dec 1990, Vol. 264, No. 21, pp. 2788-90, and Sandra Knowles in The Canadian Journal of Hospital Pharmacy, Vol. 45, No. 1, 1 Feb 1992, p.33), since this potentially lethal arrhythmia was considered to be a "class effect" among non-sedating antihistamines, in the sense that the arrhythmogenicity was considered to be coupled to the anti-histaminic potency of such compounds. Accordingly, the fact that the compositions, in accordance with the present invention, do not induce any such cardiac arrhythmias is a new, highly useful and surprising technical effect, which enables the inventive compositions to be administered to individuals susceptible to cardiac arrhythmia, and in potentially larger doses than those non-sedating anti-histamines, such as astemizole, in common use at the present time.

The anti-histaminic treatment can be a method of treating a human afflicted by or susceptible to: an allergic disorder; motion sickness; vertigo; retinopathy, or another small vessel disease associated with diabetes mellitus; cough, cold, cold-like or flu symptoms; or the discomfort, pain, fever or general malaise associated therewith. In embodiments, the invention can relate to any one, any combination, or all of the aforementioned methods of treatment.

Preferably, the invention relates to the treatment of an allergic disorder which is manifested by asthma or allergic rhinitis.

In preferred embodiments, the inventive composition further comprises a pharmaceutically acceptable carrier or excipient.

Particularly when the anti-histaminic treatment is that of cough, cold, cold-like, or flu symptoms or the discomfort, pain, fever or general malaise associated therewith, in a human, the composition can further comprise a therapeutically effective amount of a non-steroidal anti-inflammatory agent or non-narcotic analgesic, such as acetylsalicylic acid, acetaminophen, ibuprofen, ketoprofen, or naproxen, or a pharmaceutically acceptable salt thereof. Alternatively, or additionally, a composition in accordance with the present invention can further comprise a therapeutically effective amount of a decongestant, such as pseudoephedrine, or a pharmaceutically acceptable salt thereof.

In a second aspect, the invention relates to a method of providing an anti-histaminic treatment which does not induce any significant cardiac arrhythmia, to a human patient, comprising administering a therapeutically effective amount of a pharmaceutical composition in accordance with the first aspect of the invention, preferably, in one of its preferred embodiments, to said human patient. Preferably, the inventive method is a method of treating a human afflicated by or susceptible to: an allergic disorder; motion sickness; vertigo; retinopathy, or another small vessel disease associated with diabetes mellitus; cough, cold, cold-like or flu symptoms; or the discomfort, pain, fever or general malaise associated therewith. Preferably, the method is a method for treating a human afflicted by or susceptible to an allergic disorder and, more preferably, the allergic disorder is asthma or allergic rhinitis.

In a third aspect, the invention relates to a use of a composition, in accordance with the first aspect of the invention, or any of its preferred embodiments, for the manufacture of a medicament for use in an anti-histaminic treatment which does not induce any significant cardiac arrhythmia, comprising administering a therapeutically effective amount of the compound to a human patient. Preferably, said use is for the manufacture of a medicament for use in a method of treating a human afflicted by or susceptible to: an allergic disorder; motion sickness; vertigo; retinopathy, or another small vessel disease associated with diabetes mellitus; cough, cold, cold-like or flu symptoms; or the discomfort, pain, fever or general malaise associated therewith. More preferably, said method is a method of treating a human afflicted by or susceptible to an allergic disorder which, preferably, is manifested by asthma or allergic rhinitis.

Astemizole has antihistaminic activity and provides therapy and a reduction of symptoms for a variety of conditions and disorders related to allergic disorders, diabetes mellitus and other conditions; however, this drug, while offering the expectation of efficacy, causes adverse effects. Utilizing norastemizole results in diminished adverse effects, and accordingly, an improved therapeutic index. It is, therefore, more desirable to use norastemizole than to use astemizole itself. Indeed, norastemizole may be administered in greater doses than would be appropriate for astemizole.

The term "adverse effects" includes, but is not limited to, cardiac arrhythmias, cardiac conduction disturbances, appetite stimulation, weight gain, sedation, gastrointestinal distress, dry mouth, constipation, and diarrhea. The term "cardiac arrhythmias" includes, but is not limited to, ventricular tachyarrhythmias, torsades de pointes, and ventricular fibrillation.

The phrase "therapeutically effective amount" means that amount of a compound of the invention which provides a therapeutic benefit in an antihistaminic treatment, including the treatment or management of allergic disorders, asthma, retinopathy or other small vessel disorders associated with diabetes mellitus, motion sickness, vertigo, or cough, cold, cold-like, and/or flu symptoms and the discomfort, pain, fever, and general malaise associated therewith. Examples of allergic disorders include, but are not limited to, allergic rhinitis, solar urticaria, and symptomatic dermographism. The symptoms associated with these allergic disorders and the cough, cold, cold-like, and/or flu symptoms include, but are not limited to, sneezing, rhinorrhea, lacrimation, and dermal irritation. The term "asthma" is defined as a disorder characterized by increased responsiveness of the trachea and bronchi to various stimuli which results in symptoms which include wheezing, cough, and dyspnea. The term "vertigo" as used herein means the dizziness associated with, but not limited to, motion, height, and changes in body position. The term "diabetic retinopathy" or "retinopathy associated with diabetes mellitus" is that disorder caused by increased permeability of the capillaries in the eye which leads to hemorrhages and edema in the eye and can lead to blindness. The term "small vessel disorders associated with diabetes mellitus" includes, but is not limited to, diabetic retinopathy and peripheral vascular disease.

The magnitude of a prophylactic or therapeutic dose of norastemizole in the acute or chronic management of disease will vary with the severity of the condition to be treated and the route of administration. The dose, and perhaps the dose frequency, will also vary according to the age, body weight, and response of the individual patient. In general, the total daily dose range, for the conditions described herein, is from about 1 mg to about 200 mg administered in single or divided doses orally, topically, transdermally, or locally by aerosol. For example, a preferred oral daily dose range should be from about 5 mg to about 50 mg. It is further recommended that children, patients aged over 65 years, and those with impaired renal or hepatic function initially receive low doses, and that they then be titrated based on individual response(s) or blood level(s). It may be necessary to use dosages outside these ranges in some cases as will be apparent to those skilled in the art. Further, it is noted that the clinician or treating physician will know how and when to interrupt, adjust, or terminate therapy in conjunction with individual patient response.

The various terms "an amount sufficient to alleviate said allergic disorder but insufficient to cause said adverse effects," "an amount sufficient to alleviate said asthma but insufficient to cause said adverse effects," "an amount sufficient to alleviate said motion sickness but insufficient to cause said adverse effects," and "an amount sufficient to alleviate said retinopathy or other small vessel diseases associated with diabetes mellitus but insufficient to cause said adverse effects" are encompassed by the above-described dosage amounts and dose frequency schedule. In addition, the terms "a pharmaceutical composition for use in the treatment of cough, cold, cold-like and/or flu symptoms and the discomfort, pain, fever and general malaise associated therewith, in a human, said composition comprising (i) a therapeutically effective amount of norastemizole, with (ii) a therapeutically effective amount of at least one non-steroidal anti-inflammatory agent or non-narcotic analgesic" and "a pharmaceutical composition for use in the treatment of cough, cold, cold-like and/or flu symptoms and the discomfort, pain, fever and general malaise associated therewith, in a human, said composition comprising (i) a therapeutically effective amount of norastemizole, with (ii) a therapeutically effective amount of a decongestant" are also encompassed by the above-described dosage amounts and dose frequency schedule.

The pharmaceutical compositions of the present invention comprise norastemizole as the active ingredient, or a pharmaceutically acceptable salt thereof, and may also contain a pharmaceutically acceptable carrier, and optionally, other therapeutic ingredients.

The term "pharmaceutically acceptable salts" refers to salts prepared from pharmaceutically acceptable non-toxic acids or bases, including inorganic or organic acids or bases. Examples of such inorganic acids are hydrochloric, hydrobromic, hydroiodic, sulfuric, and phosphoric. Appropriate organic acids may be selected, for example, from aliphatic, aromatic, carboxylic and sulfonic classes of organic acids, examples of which are formic, acetic, propionic, succinic, glycolic, glucoronic, maleic, furoic, glutamic, benzoic, anthranilic, salicylic, phenylacetic, mandelic, embonic (pamoic), methanesulfonic, ethanesulfonic, pantothenic, benzenesulfonic, stearic, sulfanilic, algenic, and galacturonic. Examples of such inorganic bases include metallic salts made from aluminum, calcium, lithium, magnesium, potassium, sodium, and zinc. Appropriate organic bases may be selected, for example, from N,N'-dibenzylethylenediamine, chloroprocaine, choline, diethanolamine, ethylenediamine, meglumaine (N-methylglucamine), lysine and procaine.

Because of their ease of administration, tablets and capsules represent advantageous oral dosage unit forms, in which case solid pharmaceutical carriers are employed. If desired, tablets may be coated by standard aqueous or nonaqueous techniques.

In addition to the common dosage forms set out above, the compounds of the present invention may also be administered by controlled release means and/or delivery devices such as those described in U.S. Patent Nos. 3,845,770; 3,916,899; 3,536,809; 3,598,123; and 4,008,719.

Pharmaceutical compositions of the present invention suitable for oral administration are presented as discrete units such as capsules, cachets or tablets each containing a predetermined amount of the active ingredient, as a powder or granules. Such compositions may be prepared by any of the methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, surface active or dispersing agent. Molded tablets may be made by molding, in a suitable machine, a mixture of the powdered compound moistened with an inert liquid diluent. Desirably, each tablet contains from about 5 mg to about 150 mg of the active ingredient, and each cachet or capsule contains from about 5 mg to about 150 mg of the active ingredient, i.e., norastemizole. Most preferably, the tablet, cachet or capsule contains either one of three dosages, 5 mg, 10 mg or 20 mg of the active ingredient.

The invention is further defined by reference to the following examples describing in detail the preparation of the compounds and the compositions of the present invention, as well as their utility. It will be apparent to those skilled in the art that many modifications, both to materials and methods, may be practiced which are within the scope of this invention.

### EXAMPLES

### Example 1

### Preparation of Astemizole and Norastemizole

Astemizole can be synthesized by methods disclosed in U.S. Patent No. 4,219,559. Norastemizole is prepared similarly, by reaction steps conventional in the art, as depicted below:

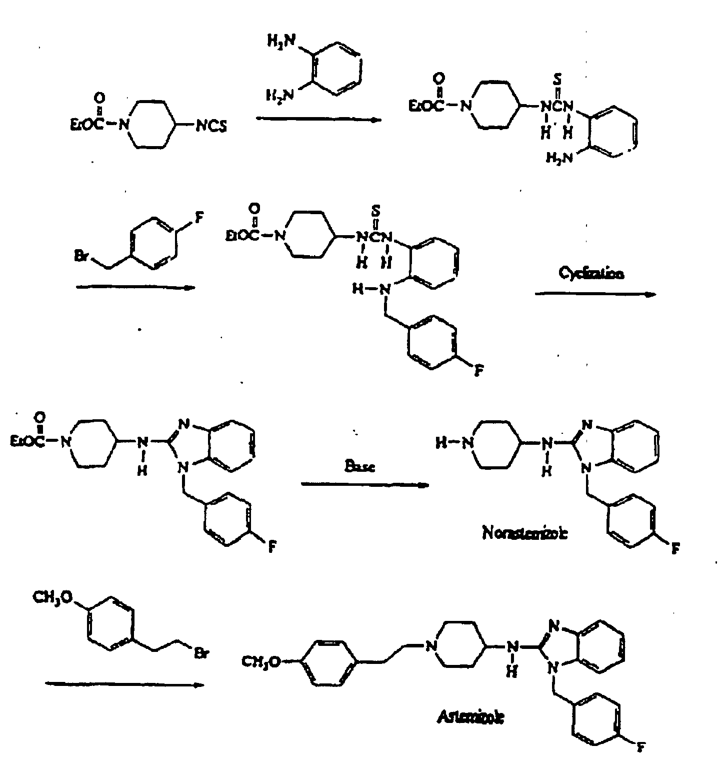

Reaction of the isothiocyanate shown with phenylenediamine gives the corresponding thiourea. N-Alkylation with p-fluorobenzylbromide gives the secondary amine which, upon cyclization, yields the substituted benzimidazole shown. Treatment of the benzimidazole with base hydrolyzes the urethane moiety to give norastemizole. N-Alkylation of norastemizole with p-methoxyphenethyl bromide yields astemizole.

### Example 2

Activities of astemizole and norastemizole at the histamine H₁-receptor were assessed using the [³H]pyrilamine binding assay as described in Chang et al., J. Neurochem. 32: 1653-1663 (1979). Briefly, membranes from bovine cerebellum were incubated with [³H]pyrilamine and varying concentrations of test compound. The reactions were carried out in 50 mM sodium phosphate buffer (pH 7.5) at 25° C for 30 minutes. The reaction was terminated by rapid vacuum filtration onto glass fiber filters. Radioactivity trapped on the filters was determined and compared to control values to acertain the interaction of the test compound with the H₁-receptor.

The metabolite (norastemizole) is approximately 100 times more potent than the parent compound (astemizole).

### Example 3

A laboratory investigation was performed to study the comparative propensities of astemizole and norastemizole to induce a form of cardiac arrhythmia, namely torsades de pointes, which results from prolongation of the cardiac action potential duration ("APD"). Such lethal arrhythmias are the most serious side effect known for astemizole.

Cats weighing 2-3 kg were anesthetized and heparinized. The heart was quickly removed through a mid-sternal thoracotomy and cannulated for retrograde perfusion in a "Langendorff apparatus." Temperature was kept at 37°C and pacing electrodes were placed in the right ventricular apex and stimulated at 400 msec cycle length. The AV node was destroyed by cautery to produce complete heart block and allow observations at slow cycle lengths. Electrodes were placed in the bath and positioned for recording of an ECG that allowed accurate measurement of the QT interval. Monophasic action potential ("MAP") catheters were placed on the left ventricular surface for recording of the duration of the cardiac action potential. All data were stored on a video recorder for later analysis.

After 20 minutes of stabilization, the QT interval and MAP duration were observed until three separate recordings (2-3 minutes apart) indicated the presence of stable measurements (<5% changes). After pre-drug recordings of QT and MAP had been obtained, the perfusion solution was changed to include astemizole or norastemizole. QT and MAP recordings were obtained continuously for at least 40 minutes at each drug concentration to obtain stable readings.

Initially, the hearts were perfused with concentrations of astemizole at 1 to 10 *µ*M, but since astemizole proved to be more potent than anticipated in prolonging APD and QT, additional experiments were run at a lower concentration (0.5 *µ*M).

Every five minutes throughout the study, the QT interval and MAP duration were measured for three cardiac cycles. Beats were selected that provided artifact-free tracings, and the QT and MAP at 90% repolarization (MAP₉₀) were recorded for each beat. The mean of the three measurements for each 5-minute period were calculated. The effects of increasing concentration on QT and MAP₉₀ were analyzed and tabulated.

The results are summarized in the following table, from which it is apparent that astemizole potently prolongs the QT interval (and APD duration). At concentrations of 0.5 to 1.0 *µ*M, astemizole prolonged the action potential duration by 20 to 25%. Norastemizole caused similar electrophysiological effects, but only at concentrations of 10 *µ*M. Thus, astemizole was 10 to 20 times more potent than norastemizole in prolonging the cardiac action potential.

| Prolongation by astemizole and norastemizole of QT interval and APD₉₀ duration | | |
|---|---|---|
| **ASTEMIZOLE** | **(Mean ± Standard Deviation)** | |
| CONC . (µM) | QT %;(N) | APD₉₀ %;(N) |
| 0.5 | 20;(1) | 25;(1) |
| 1.0 | 24 ± 5;(3) | 23 ± 5;(3) |
| 5.0 | 43 ± 19;(3) | 40 ± 12;(4) |
| 10.0 | 47;(1) | 45;(1) |

| **NORASTEMIZOLE** | **(Mean ± Standard Deviation)** | |
|---|---|---|
| CONC. (µM) | QT %;(N) | APD₉₀ %;(N) |
| 0.5 | 4;(1) | 2;(1) |
| 1.0 | 6 ± 2;(4) | 6 ± 3;(4) |
| 5.0 | 17 ± 6;(5) | 13 ± 6;(5) |
| 10.0 | 28 ± 10;(5) | 19 ± 12;(5) |

Because norastemizole was up to 100 times more potent than astemizole as an antihistaminic, while norastemizole was 10 or 20 times less potent in causing the serious arrhythmogenic side effect known to be associated with the drug, it is concluded that norastemizole has a therapeutic index on the order of up to 1000 to 2000 times higher than the therapeutic index of the parent compound.

### Example 4

| Oral Formulation - Capsules: | | | |
|---|---|---|---|
| Formula | Quantity per capsule in mg. | | |
| | A | B | C |
| Active ingredient Astemizole metabolite | 5.0 | 10.0 | 20.0 |
| | | | |
| Starch 1500 | 94.0 | 89.0 | 79.0 |
| | | | |
| Magnesium Stearate BP | 1.0 | 1.0 | 1.0 |
| | | | |
| compression Weight | 100.0 | 100.0 | 100.0 |

The active ingredient is sieved and blended with the excipients. The mixture is filled into suitably sized two-piece hard gelatin capsules using suitable machinery. Other doses may be prepared by altering the fill weight and if necessary, changing the capsule size to suit.

### Example 5

| Oral Formulation - Tablets: | | | |
|---|---|---|---|
| Formula | Quantity per Tablet in mg. | | |
| | A | B | C |
| Active ingredient, Astemizole metabolite | 5.0 | 10.0 | 20.0 |
| | | | |
| Lactose BP | 148.5 | 143.5 | 133.5 |
| | | | |
| Starch BP | 30.0 | 30.0 | 30.0 |
| | | | |
| Pregelatinized Maize Starch BP | 15.0 | 15.0 | 15.0 |
| | | | |
| Magnesium stearate | 1.5 | 1.5 | 1.5 |
| | | | |
| Compression Weight | 200.0 | 200.0 | 200.0 |

The active ingredient is sieved through a suitable sieve and blended with the lactose until a uniform blend is formed. Suitable volumes of water are added and the powders are granulated. After drying, the granules are then screened and blended with the magnesium stearate.The resulting granules are then compressed into tablets of desired shape. Tablets of other strengths may be prepared by altering the ratio of active ingredient to the excipient(s) or the compression weight.

## Claims

1. A pharmaceutical composition in the form of a tablet, cachet or capsule comprising 5, 10 or 20 mg of the compound of formula I: 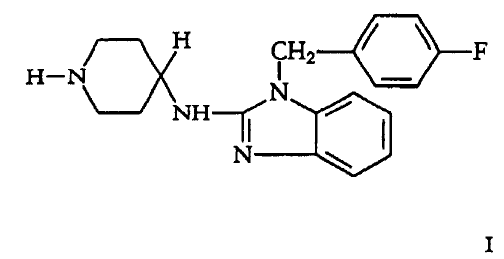 or a pharmaceutically acceptable salt thereof, for use in an anti-histaminic treatment which does not induce any significant cardiac arrhythmia, comprising administering a therapeutically effective amount of the compound of formula I to a human patient.

2. A pharmaceutical composition, as claimed in claim 1, wherein the anti-histaminic treatment is a method of treating a human afflicted by or susceptible to: an allergic disorder; motion sickness; vertigo; retinopathy or other small vessel diseases associated with diabetes mellitus; cough, cold, cold-like or flu symptoms; or the discomfort, pain, fever or general malaise associated therewith.

3. A pharmaceutical composition, as claimed in claim 2, wherein the allergic disorder is manifested by asthma or allergic rhinitis.

4. A pharmaceutical composition, as claimed in any of the preceding claims, wherein the anti-histaminic treatment comprises the administration of a compound of formula I in an amount of 1-200mg/day and, preferably, in an amount of 5-50mg/day.

5. A pharmaceutical composition, as claimed in any of the preceding claims, further comprising a pharmaceutically acceptable carrier or excipient.

6. A pharmaceutical composition, as claimed in any of the preceding claims, further comprising a therapeutically effective amount of a non-steroidal anti-inflammatory agent or a non-narcotic analgesic.

7. A pharmaceutical composition, as claimed in any of the preceding claims, further comprising a therapeutically effective amount of a decongestant.

## Patentansprüche

1. Pharmazeutische Zusammensetzung in Form einer Tablette, eines Cachet oder einer Kapsel, umfassend 5, 10 oder 20 mg der Verbindung der Formel I: 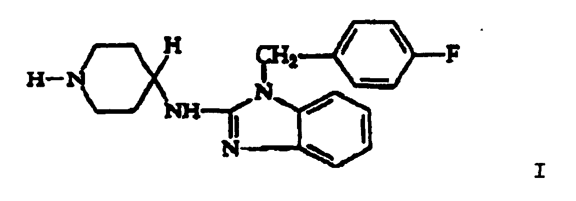 oder ein pharmazeutisch akzeptables Salz derselben, für die Verwendung bei einer Antihistaminbehandlung, die keine signifikante Herzrhythmusstörung induziert, umfassend die Verabreichung einer therapeutisch wirksamen Menge der Verbindung der Formel I einem menschlichen Patienten.

2. Pharmazeutische Zusammensetzung nach Anspruch 1, wobei die Antihistaminbehandlung eine Methode ist für das Behandeln eines Menschen, der an Folgendem leidet oder dafür anfällig ist: einem allergischen Leiden, Reisekrankheit, Vertigo, Retinopathie oder anderen mit der Zuckerkrankheit verbundenen Erkrankungen kleiner Gefäße, Husten, Erkältung oder erkältungsartigen oder Grippesymptomen oder den damit verbundenen Beschwerden, Schmerzen, Fieber oder allgemeinen Unwohlseinsgefühlen.

3. Pharmazeutische Zusammensetzung nach Anspruch 2, wobei die allergische Beschwerde sich als Asthma oder allergische Rhinitis bemerkbar macht.

4. Pharmazeutische Zusammensetzung nach einem der vorhergehenden Ansprüche, wobei die Antihistaminbehandlung die Verabreichung einer Verbindung der Formel I in einer Menge von 1 - 200 mg/Tag und bevorzugt in einer Menge von 5 - 50 mg/Tag umfasst.

5. Pharmazeutische Zusammensetzung nach einem der vorhergehenden Ansprüche, die des Weiteren einen pharmazeutisch akzeptablen Träger oder ein pharmazeutisch akzeptables Vehikel umfasst.

6. Pharmazeutische Zusammensetzung nach einem der vorhergehenden Ansprüche, die des Weiteren eine therapeutisch wirksame Menge eines nichtsteroiden entzündungshemmenden Mittels oder eines nichtnarkotischen Analgetikums umfasst.

7. Pharmazeutische Zusammensetzung nach einem der vorhergehenden Ansprüche, die des Weiteren eine therapeutisch wirksame Menge eines Dekongestionsmittels umfasst.

## Revendications

1. Une composition pharmaceutique se présentant sous la forme de comprimés, cachets ou gélules contenant 5, 10 ou 20 mg du produit de formule I : 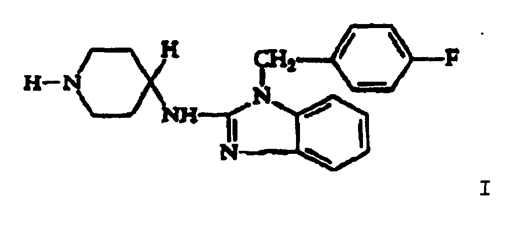 ou d'un sel pharmaceutiquement acceptable dudit composé, utilisée dans un traitement antihistaminique qui n'induit aucune arythmie cardiaque significative et comprend l'administration dudit composé de formule I à un patient humain en une quantité efficace sur le plan thérapeutique.

2. Une composition pharmaceutique selon la revendication 1, ledit traitement antihistaminique étant utilisé chez des patients humains souffrant de l'une des pathologies qui suivent ou prédisposés à l'une des pathologies en question : maladie allergique ; cinépathie ; vertige ; rétinopathie ou autre microangiopathie associée au diabète sucré ; toux, rhume, symptômes du rhume ou syndrome d'allure grippale ; ou la gêne, la douleur, l'état fébrile ou le malaise général associés à ces affections.

3. Une composition pharmaceutique selon la revendication 2, ladite maladie allergique se manifestant par un asthme ou une rhinite allergique saisonnière.

4. Une composition pharmaceutique selon l'une quelconque des revendications précédentes, ledit traitement antihistaminique comprenant l'administration d'un composé de formule I à une dose de l'ordre de 1 - 200 mg/jour, et préférablement de 5 - 50 mg/jour.

5. Une composition pharmaceutique selon l'une quelconque des revendications précédentes, qui contient également un véhicule ou un excipient pharmaceutiquement acceptable.

6. Une composition pharmaceutique selon l'une quelconque des revendications précédentes, qui contient également un anti-inflammatoire non stéroidien ou un analgésique non narcotique en une quantité efficace sur le plan thérapeutique.

7. Une composition pharmaceutique selon l'une quelconque des revendications précédentes, qui contient également un décongestionnant en une quantité efficace sur le plan thérapeutique.
